# EUROPEAN PATENT SPECIFICATION

(11) **EP 1 343 860 B1**
(45) Date of publication and mention of the grant of the patent: **25.01.2012**
(21) Application number: 01271427.5
(22) Date of filing: 20.12.2001
(51) Int. Cl.: A61K 8/92, A61K 36/00, C11B 7/00, C11C 3/10, C11C 3/12, A23D 9/00, A23D 7/00, A23L 1/30

(54) **A PROCESS FOR PREPARING VEGETABLE OIL FRACTIONS RICH IN NON-TOCOLIC, HIGH-MELTING, UNSAPONIFIABLE MATTER**
VERFAHREN ZUR HERSTELLUNG VON PFLANZENÖLFRAKTIONEN, DIE REICH AN NICHTTOCOLHALTIGEM, HOCHSCHMELZENDEM, UNVERSEIFBAREM MATERIAL SIND
PROCEDE POUR LA PREPARATION DE FRACTIONS D'HUILE VEGETALE RICHES EN MATIERE INSAPONIFIABLE, NON TOCOLIQUE ET A POINT DE FUSION ELEVE

(30) Priority: 21.12.2000 DK 200001917; 09.01.2001 US 260591 P
(43) Date of publication of application: 17.09.2003
(73) Proprietor: AarhusKarlshamn Denmark A/S, 8000 Århus C (DK)
(72) Inventor: MELLERUP, Jens, DK-8381 Tilst (DK); BACH, Mogens, DK-8000 Aarhus C (DK); ENKELUND, J rgen, Valentin, DK-8220 Brabrand (DK)
(74) Representative: Olsen, Lars Pallisgaard
(86) International application number: PCT/DK2001/000849
(87) International publication number: WO 2002/050221

(56) References cited:
- EP-A- 1 001 007
- WO-A-01/70046
- WO-A-96/03137
- WO-A-96/38047
- WO-A-97/00309
- WO-A-99/63031
- GB-A- 2 023 635
- SINGLETON W S ET AL: "TOCOPHEROL CONCENTRATES BY THE FRACTIONAL CRYSTALLIZATION OF COTTONSEED OIL FROM SOLVENTS" OIL AND SOAP, AMERICAN OIL CHEMISTS'SOCIETY. CHAMPAIGN, US, vol. 21, 1944, pages 224-226, XP001015629
- WESTSTRATE J A ET AL: "PLANT STEROL-ENRICHED MARGARINES AND REDUCTION OF PLASMA TOTAL-AND LDL-CHOLESTEROL CONCENTRATIONS IN NORMOCHOLESTEROLAEMIC AND MILDLY HYPERCHOLESTEROLAEMIC SUBJECTS" EUROPEAN JOURNAL OF CLINICAL NUTRITION, XX, XX, vol. 52, no. 5, May 1998 (1998-05), pages 334-343, XP000884738 cited in the application
- SIERKSMA A ET AL: "SPREADS ENRICHED WITH PLANT STEROLS, EITHER ESTERIFIED 4,4-DIMETHYLSTEROLS OR FREE 4-DESMETHYLSTEROLS, AND PLASMA TOTAL- AND LDL-CHOLESTEROL CONCENTRATIONS" BRITISH JOURNAL OF NUTRITION, CAMBRIDGE, GB, vol. 82, 1999, pages 273-282, XP001015683 cited in the application
- DATABASE WPI Section Ch, Week 199737 Derwent Publications Ltd., London, GB; Class B05, AN 1997-399453 XP002175063 -& JP 09 176057 A (KOYO FINE CHEM KK), 8 July 1997 (1997-07-08)

## Description

The present invention refers to a new process for the fractionation of vegetable oils and fats which gives a fraction highly enriched in non-tocolic, high-melting, unsaponifiable matter. Basically the process does not generate trans-fatty acids. The oil fractions can be tailored to specific applications as claimed.

### BACKGROUND

Vegetable oils and fats are mainly triglycerides and other saponifiable matter such as mono and diglycerides and traces of free fatty acids. Besides these substances they have a varying content and composition of unsaponifiable constituents.

The unsaponifiable matter (or unsaponifiables) is the material which can be extracted by petroleum ether or other similar solvent after alkaline hydrolysis of a sample. The unsaponifiable constituents and the relative composition are typical for the individual oil. A large number are biologically active components, and the following listing is not exhaustive, but it reflects their importance when it comes to documented functionality, as well as their main sources:

### 1. Tocols

The constituents are tocopherols and tocotrienols.

Tocopherols are present in practically all vegetable oils. Soybean, corn, sunflower and rapeseed oils are the main commercial sources. Tocotrienols are mainly present in palm, rice bran, barley and wheat germ oil. Commercial quantities of tocotrienols are sourced from palm and rice bran oil.

Tocopherols are nature's major lipid-soluble antioxidant and are often referred to as natural vitamin E (d-α-tocopherol has the highest biopotency, and its activity is the standard against which all the others are compared). Vitamin E is an in vivo antioxidant protecting cell membranes against the damaging effects of free radicals. The application as a supplement and an additive in food and feed is well established. The vast data available also point to its importance in formulating functional cosmetics.

Tocotrienols are related to tocopherols and are considered to be powerful antioxidants. Some studies suggest that the antioxidant potential is even greater than that of the tocopherols. Topical application of tocotrienols has also been found helpful in fighting oxidative damage to the skin while preserving the existing vitamin E in skin cells.

### 2. Phytosterols

The major constituents are β-sitosterol, campesterol and stigmasterol. Normally they account for more than 70% relative of the sterol fraction. Minor, normally occurring sterols are: stigmastenol, avenasterols and cholesterol.

Sterols are present in almost all vegetable oils. Commercial quantities are mainly sourced from soybean oil. Selection criterions are availability, total content and composition. As an example, rapeseed oil would be a good choice if brassicasterol was wanted as a constituent.

Topical application of a preparation that contains phytosterols results in an increase in both moisture in the skin and lipid content on the skin. The phytosterols adhering to the surface of the skin impart a hydrophobic surface, which is not reversed after exposure to water and soap. There are several studies reported in literature on the beneficial effect of sterols in restoring skin irritation which originates from external sources, i.e. UV-radiation, detergents, wet nappy exposure, etc.

Nutritional compositions containing phytosterols, mainly β-sitosterol and stannol esters, are reported to have a cholesterol-lowering action. The mechanism is not fully understood, but the plant sterols appear to inhibit the uptake of cholesterol from the gut. The effect is that both total and LDL cholesterol level in the blood are reduced.

Sterols are furthermore useful in the pharmaceutical industry for conversion into steroid derivatives.

### 3. Triterpene Alcohols (TTPA)

The major constituents are α- and β-amyrin, butyrospermol and lupeol. They are mainly present in the form of cinnamic, acetic and fatty acid esters.

The main source is shea butter, but TTPAs are also present in illipe, sal and shorea butter.

For centuries, shea butter has been used traditionally on the African continent for its outstanding properties in protecting and restoring the skin. This is especially attributed to the unsaponifiable matter. A number of tests on human volunteers have proven a series of effects on various kinds of skin problems: cicatrising action in treatment of chapping, restoring dermatitis and erythema from sunburn, etc. In animal tests the butyrospermol fraction is reported to have a cortisone-like action on oral and parenteral administration.

### 4. γ-Oryzanol

γ-Oryzanol is generally assigned to ferulic acid esters of steroid moieties. The main steroids are β-sitosterol, campesterol, cycloartenol and 24-methylcycloartenol.

γ-Oryzanol is found in rice germ and rice bran oil. It is reported to have growth-accelerating effect on animals. Topical application of an ointment which contains γ-oryzanol stimulates the blood flow in the peripheral veins. In tests on human volunteers it is reported to have a cholesterol-lowering effect on both total and LDL cholesterol level.

### 5. Carotenoids

Carotenoids are mainly present in palm oil. The oil of Elaeis oleifera has the highest content of mixed natural carotenoids.

The major constituents are α- and β-carotene.

β-carotene is providing vitamin A activity. It is reported to possess anti-cancer properties for certain types of cancer, but in some investigations α-carotene is found to be more potent. It is also worth noting that other carotenoids present in palm oil, phytolene and especially lycopene, have been reported to possess even better anti-cancer properties.

The mixed natural carotenoids can furthermore be used as a natural colouring agent in various applications.

Besides the classes of unsaponifiables listed above, there are many other types of interest where the sourcing has to be evaluated according to the relative presence and total concentration in the oil. This is illustrated by the following examples: Olive oil has an interesting content of the triterpene polyunsaturated aliphatic hydrocarbon, squalene. In avocado oil approx. 50% of the unsaponifiable matter consists of alkylfuranes. Sesame seed oil contains sesamolin and sesamin.

All in all, this clearly demonstrates the need for industrial processes tailored to enrich and standardise these valuable substances making it possible to direct and dose them in a proper manner for the application in question.

### STATE OF THE ART

In general the concentration of unsaponifiable matter is low in the raw material. Consequently, industrial fatty residues, normally considered as waste, are in use as starting material. An example of this is the production of phytosterols (mainly sitosterols) based on wood-derived by-products.

By the processing of vegetable oils and fats huge quantities of waste are generated i.e. soap stock by the alkaline refining method and deodoriser distillate. Deodoriser distillate is of particular interest as it contains 10-30 % unsaponifiable matter of which approximately half is tocopherols and sterols. The separation and purification of the tocopherols and sterols is a complex and expensive process involving physical and chemical methods. The commercial products available are based mainly on deodoriser distillate from soybean oil.

Vegetable oils are used as the starting material in a process that involves either saponification of the oil or conversion of the glycerides into fatty acid methyl esters followed by extraction and purification of the unsaponifiable matter. The commercial products are tocopherol/tocotrienol mixtures and natural mixed carotenoids based on rice bran or palm oil.

The processes described so far are in use on an industrial scale. The following processes are directly applicable to oils and fats and mainly related to sheanut oil as the starting material. Sheanut oil unsaponifiables have interesting, documented dermatological properties, and the oil has a beneficial high non-glyceride content of approx. 6 %.

In WO 96/03137 it is illustrated that a sheanut oil fraction with 10 % unsaponifiable matter can be enriched to 50% by short-path distillation.

By applying supercritical CO₂ extraction on sheanut oil it is experimentally demonstrated that a selective fractionation of the constituents is possible at temperatures of 40-80 °C and pressures of 100-400 bar (Turpin et al. 1990. *Fat Sci. Technol.* **92 (5)**, 179-184).

Another method for the production of lipid fractions enriched in unsaponifiables is by fractional crystallisation from solvents.

It is experimentally demonstrated that tocopherol concentrates can be obtained from vegetable oils by almost completely removing the glycerides and sterols by fractional crystallisation from solvents at low temperatures. As an example, partially hydrogenated cottonseed oil yielded a concentrate containing 32.1% tocopherols by crystallisation of the oil from 8 times its own weight of acetone at -74 °C. Acetone proved to be the most suitable solvent (Singleton and Bailey 1944. oil & Soap 21, 224-226).

EP 0690904 teaches how to prepare two fractions rich in unsaponifiable matter from shea butter. The fat is treated with a polar solvent of the ketone type in order to recover one fraction insoluble in the hot ketone solvent (polyisoprenic hydrocarbons). The hot ketone solvent is cooled to a temperature below 0 °C to crystallise the hot soluble material, which is filtered off. The filtrate is desolventised giving a second fraction rich in unsaponifiable matter (sterols and TTPA-esters). The preferred solvent is acetone. The two fractions are mixed at least in part or preferably in total to give a mixture claimed to contain 18-50% by weight of the unsaponifiable matter especially useful in dermatological compositions.

WO 99/63031 teaches how to fractionate a vegetable oil which gives one or more solid fractions suitable for confectionery applications as well as a liquid fraction enriched in unsaponifiable matter. The oil to be fractionated must have a slip melting point of 32-55 °C and, if necessary, the oil is partly hydrogenated to reach said melting interval. In the description page 9, line 2 from the bottom, it is stated that if the slip melting point of the oil is more than 55 °C, the amount of liquid to solubilise the desired components will be too small and they will be precipitating together with the solid triglycerides, and further, that the recovery of the small amount of the liquid fraction will also be more difficult than if a lower slip melting point is used. The fat is mixed with a solvent (acetone is preferred) and heated to transparency and cooled to precipitate a solid fraction. The filtrate is desolventised to give a liquid fraction in which the unsaponifiable matter is enriched. The process is exemplified in a rapeseed fraction claimed to contain 1.5-5% unsaponifiable components, useful as an ingredient in cosmetic and pharmaceutical preparations for providing moisturising, UV-protecting and antiinflammatory properties. The process is further exemplified in a shea butter fraction claimed to contain 15-36% unsaponifiable components, useful as an ingredient in cosmetic and pharmaceutical preparations for providing UV-protection and skin moisturising properties.

To summarise the following can be stated:
The use of waste material, especially the deodoriser distillate, has proven that it is possible to produce tocopherols and phytosterols of high purity. The process is complex and expensive but driven by the high prices of tocopherols. The main problem is the sourcing of the starting material, as vegetable oil production is scattered.

The extraction and purification via the methylester or saponification route is expensive and leaves high amounts of cheap by-products i.e. fatty acids, glycerine and methyl esters. The commercial products have a content of 30-50% unsaponifiable matter.

The direct processing from oils and fats by short-path distillation and supercritical fluid extraction is at present on an experimental stage.

The use of dry or solvent fractional crystallisation has limitations. If the concentration of unsaponifiable matter is low in the oil, the resulting enrichment is also low. Furthermore, if the starting material is liquid, it has to be partly hydrogenated before fractionation. The trans-fatty acids formed during partial hydrogenation of the unsaturated fatty acids in the glycerides may increase the blood cholesterol level and the risk of coronary heart disease if eaten. This makes the enriched fractions unsuited for the fortification of food and as a nutritional supplement or a pharmaceutical or parma-like product for oral administration.

### OBJECT OF THE INVENTION

The invention aims at providing a process for preparing vegetable, oil fractions highly enriched in non-tocolic, high-melting, unsaponifiable matter by which the enrichment of the higher melting unsaponifiables (e.g. phytosterols, triterpene alcohols and esters, γ-oryzanol, carotenoids, etc.) and the composition of the glyceridic part of the oil fraction can be tailored to the specific application. Basically the process should not generate trans-fatty acids.

### SUMMARY OF THE INVENTION

This is achieved by the process of the invention which comprises the following steps:
a) a vegetable oil, or a fraction thereof, having a slip melting point of not more than 30 °C and a content of unsaponifiable matter of at least 0.5 % by weight is hydrogenated to fully saturate the fatty acids of the glycerides and to reach a slip melting point of at least 57 °C;
b) to the hydrogenated oil is added from 1 to 75 % by weight of the unhydrogenated starting oil or another oil having a slip melting point of max. 30 °C to act as a carrier and vehicle for the unsaponifiable matter;
c) to the oil mixture from b) is added a solvent in a ratio from 1:2 to 1:20, and the mixture is heated to transparency;
d) the mixture from c) is cooled in one or more steps to a final temperature in the range from -35 to +30 °C, and the precipitated high-melting fraction(s) is (are) filtered off;
e) the filtrate obtained from d) is desolventised, leaving a fraction rich in unsaponifiable matter.

By the hydrogenation in step a) besides the unsaturated fatty acid moieties also some of the unsaponifiables containing double bonds are converted to the corresponding hydrogenated forms; especially in triterpene alcohols double bonds in side chains will be hydrogenated.

Often it is desirable to conduct step b) by adding from 1 to 75 % by weight, preferably from 2 to 50 % by weight, and more preferably from 5 to 25 % by weight, of the unhydrogenated starting oil as a carrier to the hydrogenated oil from step a). This makes it possible to adjust the content of unsaponifiable matter in the resulting enriched fraction and, at the same time, to keep the trans-fatty acid content of the enriched fraction at maximum 2 %.

Another way of conducting step b) is by adding from 1 to 75 % by weight, preferably from 2 to 50 % by weight, and more preferably from 5 to 25 % by weight, of a triglyceride oil different from the unhydrogenated starting oil as a carrier to the hydrogenated oil from step a), said triglyceride oil predominantly consisting of triglycerides of saturated and unsaturated C8-C22 fatty acids, and preferably being a vegetable oil predominantly consisting of triglycerides of saturated and unsaturated C16-C22 fatty acids. Hereby, it is possible to select a carrier oil having specific beneficial effects in combination with the unsaponifiable matter for the intended use.

Further, it may be desirable that the starting oil containing the unsaponifiable matter is interesterified before the hydrogenation step and/or that the resulting oil mixture is interesterified before the fractionation steps c) and d).

Normally, the solvent used in the fractionation steps c) and d) is of a non-polar or semi-polar type, e.g. selected from the group consisting of hydrocarbons and linear and branched alkanols of 1-5 carbon atoms; and it is preferably hexane or petroleum ether.

Suitably, the starting vegetable oil containing the unsaponifiable matter originates from one of the following or their hybrids: rapeseed, canola, soybean, corn, maize-germ, sunflower, flax (low-linolenic linseed), mango, avocado, olive, sesame, rice bran, wheat germ, oat and oat bran, palm, sal, shorea, illipe and shea, as well as any fraction or mixture thereof.

A starting oil of particular interest is a dekaritenised, lower-melting fraction of shea butter enriched in unsaponifiable matter, which is obtained as follows:
The crude shea butter is deacidified and subsequently partly dekaritenised by mixing it with a semi-polar solvent to precipitate the major part of the polyisoprenic hydrocarbons (karitene) which are disposed of; the solvent is distilled off and the oil is mixed with a suitable solvent e.g. hexane (ratio approx. 1:2 to 1;4), heated to transparency, and cooled to a temperature at which the high-melting glycerides precipitate; the precipitated fraction is filtered off and the filtrate desolventised leaving a lower-melting fraction with a slip melting point of max. 30 °C suitable for further processing.

Generally, the vegetable oil fraction enriched in unsaponifiable matter obtained by the process of the invention may be subjected to further concentration by means of supercritic carbondioxide, molecular distillation, chromatography or recrystallisation and/or to chemical modification such as hydrogenation, ethoxylation, esterification or interesterification without leaving the inventive concept. Also, the physical state of the vegetable oil fraction obtained may further be changed e.g. by emulsification, admixing with a high-melting fat and spray cooling, encapsulation or incorporation in liposomes or nanosomes.

The present invention also concerns a vegetable oil fraction obtainable by the process according to the invention, said fraction being enriched in non-tocolic, high-melting, unsaponifiable matter by a factor of at least 3 and having a trans-fatty acid content of maximum 2% by weight and the vegetable oil fraction for use as an ingredient in pharmaceuticals or pharma-like products for topical application or oral administration.

In such vegetable oil fraction the unsaponifiable matter is predominantly comprised of or derived from:
- carotenoids;
- sesamin and sesamolin;
- γ-oryzanol;
- sterols, methyl sterols and dimethyl sterols;
- triterpene alcohols and their esters with cinnamic acid, acetic acid and fatty acids.

The invention further relates to a particular enriched fraction of shea butter obtainable by the process according to the invention having a content of at least 30 % by weight of non-tocolic, high-melting, unsaponifiable matter and being further characterised by containing hydrogenated triterpene alcohols and/or their esters and by having a trans-fatty acid content of maximum 2 % by weight. Preferably, such shea butter fraction has a content of 40-90 % by weight of unsaponifiable matter. Further, it may be desirable that the preparation of such shea butter fraction has involved at least one interesterification step before the fractionation steps c) and d).

The present invention further comprises the non-therapeutical use of a vegetable oil fraction according to the invention for the fortification of food products.

Thus, the invention comprises the use of a vegetable oil fraction according to the invention as a constituent of a dairy or dairy-like food product selected from the group consisting of milk, cream, ice cream, butter, cheese, including soft, cream and processed cheeses, yoghurt and other fermented milk products.

It also comprises the use of a vegetable oil fraction according to the invention as a constituent of a food product selected from the group consisting of margarines, butters and blends, spreads, mayonnaise, shortenings, dressings and salad oils at a concentration of 2-50% by weight.

Further, the invention comprises the non-therapeutical use of a vegetable oil fraction according to the invention as an ingredient in cosmetics.

In particular, the invention comprises the non-therapeutical use of a vegetable oil fraction according to the invention as an ingredient in a nutritional supplement for oral administration. Specifically, it may be used as an additive to fat-containing food products or as an additive to oils and speciality fats to be incorporated in food products for humans and other mammals with the purpose of lowering the blood cholesterol level.

Finally, the invention comprises the use of a shea butter vegetable oil, fraction according to the invention for the manufacture of a medicament for lowering the blood cholesterol level.

### DETAILED DESCRIPTION OF THE INVENTION

The starting oil is obtained by means of standard crushing, pressing and extraction techniques, optionally followed by acid water treatment, deacidification and bleaching steps normally used in the processing of vegetable oils. If the starting oil has a slip melting point higher than 30 °C the oil is dry or solvent fractionated to obtain a lower melting fraction suitable as a starting oil for the process. The starting oil can also be a mixture of oils or fractions of oils obtained from different sources of raw materials. The prerequisite is that the starting oil has a slip melting point of not more than 30 °C and an unsaponifiable content of at least 0.5 % by weight.
I. In a first aspect the present invention relates to a process for preparing an oil fraction rich in non-tocolic, high-melting, unsaponifiable matter which comprises the following steps:
a) the starting oil is hydrogenated to fully saturate the fatty acids of the glycerides and to reach a slip melting point of at least 57 °C;
b) to the hydrogenated oil from a) is added a suitable amount of the starting oil to act as a carrier and vehicle for the unsaponifiable matter. The concentration of the carrier oil in the oil mixture is 1-75 % by weight, preferably 2-50 % by weight, and more preferably 5-25 % by weight;
c) to the oil mixture from b) is added a solvent in a ratio from 1:2 to 1:20, preferably from 1:3 to 1:15, and the mixture is heated to transparency;
d) the mixture from c) is cooled in one or more steps to a final temperature in the range from - 35 to +30 °C, preferably from -20 to +20 °C, and the precipitated high melting fraction(s) is (are) filtered off;
e) the filtrate obtained from d) is desolventised, leaving a fraction enriched in unsaponifiable matter.

Suitable conditions for the hydrogenation under a) are illustrated in the examples, but other variations are possible and within the ability of a person skilled in the art of processing vegetable oils.
The amount of starting oil to be added in step b) is dependent upon the original content of unsaponifiable matter in the starting oil and the type of unsaponifiable matter as well as the final concentration of unsaponifiable matter needed for the application in question. The concentration in the enriched fraction and its melting behaviour is regulated by varying the mixture ratio of carrier oil to the hydrogenated oil. This is further illustrated in the examples.
The solvent used in c) is of a non-polar or semi-polar type such as hydrocarbons and linear and branched alkanols of 1-5 carbon atoms. A preferred solvent in the process is hexane or petroleum ether.
In the fractionation step of d) one would expect the higher melting unsaponifiables to be entrapped and coprecipitated with the high melting glycerides, especially if the amount of liquid carrier oil in the oil mixture is low. Quite unexpectedly the major part of the total mass of high-melting unsaponifiables is recovered.
The oil fraction prepared by this first aspect of the invention is characterised by having a trans-fatty acid content of maximum 2 % by weight.
The enriched fraction can be further purified by deacidification, bleaching and deodorisation in the normal manner. During deodorisation a part of the tocopherols etc. are stripped off and, therefore, antioxidants and other stabilisers may optionally be added to the product. If the product is liquid or semi-liquid, addition of a structuring fat or wax is useful in order to prevent sudden precipitation of supersaturated unsaponifiables in the product.
II. In a second aspect the present invention relates to a process as detailed under heading I, except that the carrier oil added to the fully hydrogenated starting oil in step b) is different from the starting oil.
The carrier oil used in this aspect is selected to incorporate beneficial effects in combination with the unsaponifiable matter for the intended use, e.g. nutritional aspects such as the degree and type of unsaturation of the fatty acid residues of the oil, and oxidative resistance, etc. The selected carrier oil predominantly consists of triglycerides of saturated and unsaturated C8-C22 fatty acids, and has a slip melting point of not more than 30 °C. Preferably the selected carrier oil is a vegetable oil predominantly consisting of triglycerides of saturated and unsaturated C16-C22 fatty acids.
If the selected carrier oil is obtained from unhydrogenated material, then the resulting enriched oil fraction will have a trans-fatty acid content of maximum 2 % by weight. If the carrier oil is the liquid fraction of a partly hydrogenated oil, the resulting enriched oil fraction may have a trans-fatty acid content exceeding 2 % by weight.
The magnitude of enrichment and the total content of unsaponifiable matter obtainable by the procedures under I and II are illustrated in the examples and summarised in the following table.

| **Subject** | **Example 1 Rapeseed** | **Example 2 Rapeseed** | **Example 3 Shea Butter** | **Example 3 Shea Butter** |
|---|---|---|---|---|
| Unsaponifiable content in starting oil, in % | 0.9 | 0.9 | 12.5 | 12.5 |
| Carrier oil content in the mixture, in % | 2 | 5 | 10 | 20 |
| Yield of the enriched fraction, in% | 5.9 | 8.2 | 21.0 | 29.0 |
| Unsaponifiable content in enriched fraction, in% | 14.5 | 10.9 | 53.0 | 38.1 |
| Enrichment factor | 16.1 | 12.1 | 4.2 | 3.0 |
| Recovery of unsaponifiable matter, in% | 94.4 | 98.4 | 89.0 | 88.4 |

III. In a third aspect the invention relates to a process as detailed under headings I or II, wherein the starting oil containing the unsaponifiable matter is interesterified before the hydrogenation step a) and/or the oil mixture from step b) is interesterified before the fractionation steps.
The interesterification of the starting oil increases the oil solubility of a part of the unsaponifiables, and the interesterification of the oil mixture randomises the fatty acid content and thus incorporates unsaturated fatty acids in the glyceridic part of the resulting enriched oil fraction.
The interesterification is performed in the presence of a suitable catalyst, e.g. methoxide, ethoxide, or a mixture of glycerol and caustic lye, as illustrated in the examples, but other variations are possible and within the ability of a person skilled in the art of processing vegetable oils.
As mentioned in the beginning, any vegetable oil or fraction thereof is suited to be processed according to the invention provided that it has a slip melting point of not more than 30 °C and an unsaponifiable content of at least 0.5 % by weight. Preferred starting oils for the process originate from the following or their hybrids: rapeseed and canola (Brassica napus, campestris etc.), soybean (Glycine max), corn (maize) germ (Zea mays), sunflower (Helianthus annuus), flax (Lineum usitatissimus), mango (Mangifera indica), avocado (Persea americana), olive (Olea europea), sesame (Sesamum indicum), rice-bran (Oryza sativa), wheat germ (Triticum aestivum), oat and oat bran (Avena sativa), palm (Elaeis guineensis, oleifera etc.), sal (Shorea robusta), illipe (Madhuca spp.), shorea (Shorea stenoptera), sheanut (Butyrospermum parkii) or fractions or mixtures thereof.
Sheanut oil (shea butter) is of particular interest due the nature of the unsaponifiable constituents and a total content of approx. 6% thereof in the shea butter. As the slip melting point of shea butter is normally higher than 30 °C, a liquid fraction has to be used as the starting oil. The liquid fraction of shea butter is a result of the production of the high melting fraction known in the industry under the INN-designation "Shea Stearine". The preferred liquid fraction has a further benefit as it has a content of 8% unsaponifiables or higher.

A more preferred fraction is obtained by the fractionation of a partly dekaritenised shea butter. This fraction is especially suitable, as the polyisoprenic hydrocarbons (karitene) are unwanted in edible products. Furthermore, as the karitene is highly unsaturated it thereby gives rise to problems associated with oxidation. The following is an illustration of the typical relative composition of the main types of unsaponifiables in shea butter and the liquid fractions:

| **Types of unsaponifiable** | **Shea butter** | **Liquid fraction of shea butter** | **Liquid fraction of dekaritenised shea butter** |
|---|---|---|---|
| TTPAs and esters | 65 % | 70% | 85 % |
| Phytosterols and others | 6% | 6% | 8% |
| Karitene | 29 % | 24 % | 7% |

On the basis of the liquid fractions of shea butter the invented process makes it possible to manufacture enriched fractions with an unsaponifiable content in the range 30 - 90% by the fractionation of oil mixtures containing up to 50% by weight of the unhydrogenated shea butter fraction, that acts as the carrier oil for the unsaponifiable matter. Fractionation of an oil mixture containing up to 75 % by weight of the unhydrogenated shea butter fraction will result in an enriched fraction with an unsaponifiable content of 20 % by weight which corresponds to an enrichment factor of at least 3 vs. the normal content in shea butter.

### POSSIBLE USES OF THE ENRICHED FRACTIONS OBTAINED BY THE PROCESS

Some of the obvious applications are already illustrated in the section BACKGROUND above.

Due to the high concentrations of unsaponifiables obtainable by the process of the invention the enriched fractions can be used as a starting material for further mechanical and/or chemical processing.

Some new and special applications are related to fractions made on the basis of sheanut oil.

It is known from published studies (Weststrate and Meijer 1998. Eur. J. Clin. Nutr. 52 (5) : 334-343; Sierksma, Weststrate and Meijer 1999. Brit. J. Nutr. 82:273-282; Vissers, Zock, Meijer and Katan 2000. Am. J. Clin. Nutr. 72:1510-1515) that the naturally occurring unsaponifiable matter in sheanut oil is not suited for lowering cholesterol levels in serum. Surprisingly it has been found that shea butter fractions made according to the process of the present invention are able to reduce the total and LDL cholesterol level in the human blood. The results are detailed in Example 9.

As a result of this, a fraction of shea butter manufactured according to the invention can be used in food products and nutritional supplements, and due to the high concentrations obtainable by the process it can be used as an active ingredient in capsules and tablets for oral administration for the purposes illustrated in Example 9.

The invention will be better understood with reference to the following examples that are illustrative and should not be taken as limiting the scope of the present invention as described in the claims.

### EXAMPLES

### Methods of analysis and definitions

Total unsaponifiable matter (< 15%), determined according to DGF C-III Ib

Total unsaponifiable matter (> 15%), is determined as 100% minus the total glyceridic content. The glyceridic content is calculated on the basis of the actual content of fatty acids in the sample analysed by absolute quantitative GC according to IUPAC 2.301 and 2.304.

Karitene is determined by methylating the sample with 0.5n KOH in methanol. The remanence is washed with hot methanol, dissolved in chloroform and the content determined gravimetrically.

TTPA, hydrogenated TTPA and esters thereof are analysed by GC according to a modified IUPAC 2.323 using cholesteryl palmitate as internal standard.

Slip melting point is determined according to AOCS Cc 3-25.

SFC (solid fat content) is analysed by puls-NMR according to IUPAC 2.150.

Fatty acids are analysed by GC according to IUPAC 2.301 and 2.304.

Total content of tocopherols is determined by HPLC with 0.6% pentanol in hexane as mobile phase and with fluorescence detection (extinction 291 nm, emission 330 nm) vs. a reference sample.

Acid value is determined according to IUPAC 2.201.

Iodine value is determined according to IUPAC 2.205.

Enrichment factor is calculated as the fraction: content of unsaponifiables in the enriched fraction divided by the content in the starting oil.

"bdl" denotes: "below detection limit".

"Transparent" means that the liquid is allowing light to pass through so that objects behind can be clearly seen, i.e. more clear than translucent.

### Example 1. Fraction of rapeseed oil enriched in unsaponifiable content

A semi-refined low-erucic rapeseed oil with an iodine value of 115.7 is fully hydrogenated to an iodine value of 1.3 and a slip melting point of 68.0 °C. Rapeseed oil is added to the hydrogenated oil to a content of 2% by weight. The oil mixture is prepared by adding 20 g of rapeseed oil to 980 g of melted hydrogenated oil to reach a total weight of 1000 g.

In a jacketed vessel equipped with a stirrer 10 litre of hexane is added to the oil mixture and the suspension is heated to transparency. From a temperature of approx. 50 °C the mixture is cooled at a rate of approx. 1 °C/min to reach a final temperature of 5 °C. The precipitated high-melting fraction is filtered off and washed with hexane. The high-melting fraction has an iodine value of 0.5 and a slip melting point of 68.7 °C.

The filtrate is desolventised leaving an enriched fraction in a yield of 58.5 g corresponding to 5.9 % by weight of the oil mixture.

| Parameter | Starting oil | Enriched fraction |
|---|---|---|
| Total unsaponifiables, % | 0.9 | 14.5 |
| Total tocopherols, ppm | 698 | 5593 |
| Total trans-fatty acids, % | bdl | 1.0 |
| Enrichment factor calc. on total unsaponifiables | | 16.1 |
| Enrichment factor calc. on total tocopherols | | 8.0 |

Based on the analytical figures enrichment factors have been calculated. This clearly demonstrates that by applying this new process of the invention on an oil with a low content of unsaponifiable matter it is possible to produce a highly enriched fraction that at the same time has a low trans-fatty acid content making it suitable for food and non-food applications.

The enrichment factor for total unsaponifiables is much higher than for the low melting tocopherols.

Furthermore, the invented process is recovering the major part of the total unsaponifiable matter. The total mass in the oil mixture is 9 g (0.9 % of 1000 g), and the yielded fraction contains 8.5 g (14.5 % of 58.5 g) corresponding to a recovery of 94.4 % of the total unsaponifiable matter.

### Example 2. Oil enriched with unsaponifiable matter originating from rapeseed

The procedure in Example 1 was repeated, except that the carrier oil used was a speciality oil with a high oxidative resistance. This oil is commercially available under the trade name "Cremeol PS-6" (Cremeol is a trademark of Aarhus Olie). The oil is produced by multiple dry-fractionation of partly hydrogenated rapeseed, sunflower or soybean oil.

The oil is characterised by the following typical values:

| | |
|---|---|
| Saponification value | 186-195 |
| Iodine value | 82-92 |
| Slip melting point, °C | 6 |
| Solid fat content at 20 °C, % | 0 |
| Rancimat value at 120 °C, h | 40 |
| Predicted shelf life at 20 °C, years | 5 |
| Fatty acids by GC: | |
| Palmitic, C16:0, % | 4 |
| Stearic, C18:0, % | 4 |
| Total C18:1, % | 84 |
| Linoleic, C18:2, % | 5 |
| Total trans-fatty acids, % | 29 |

The carrier oil "Cremeol PS-6" was added to the fully hydrogenated rapeseed oil in an amount of 5% of the total oil mixture. The fractionation of the oil mixture was performed as described in Example 1.

The results are described in the following table.

| Parameter | Starting Oil | Enriched Fraction |
|---|---|---|
| Total unsaponifiables, % | 0.9 | 10.9 |
| Total Tocopherols, ppm | 698 | 5322 |
| Total trans-fatty acids, % | bdl | 22.8 |
| Enrichment factor calc. | | |
| on total unsaponifiables | | 12.1 |
| Enrichment factor calc. | | |
| on total tocopherols | | 7.6 |
| Yield of fraction in g | | 82 |
| Recovered amount of | | |
| unsaponifiables in g | | 8.9 |
| Recovery in % of total matter | | 98.9 |

The enriched fraction appeared to have a consistency similar to petroleum jelly. The slip melting point was 44.9 °C.

This clearly demonstrates that it is possible to enrich a suitable carrier oil with the unsaponifiable matter in question by applying this new process of the invention. Like in Example 1 the enrichement factor for total unsaponifiables is much higher than for the low melting tocopherols.

As can be seen, the process does not generate trans-fatty acids. The content of trans-fatty acids in the fraction is inherent to the preferred carrier oil.

Due to the high oxidative resistance provided by the carrier oil the produced fraction is ideally suited as an ingredient in cosmetics, toiletries and pharma products for dermal application.

### Example 3. Fraction of sheanut oil enriched in unsaponifiable matter

The starting oil in this example is an industrially produced, semi-refined and partly dekaritenised fraction of shea butter obtained by the following processing steps commonly used in the vegetable oil industry for the production of food ingredients:
1. The crude oil is obtained by means of the standard crushing, pressing and extraction techniques.
2. The free fatty acids are removed in an alkaline deacidification process followed by a bleaching step to reduce the colour.
3. The semi-refined oil is dekaritenised by mixing it with a semi-polar solvent by which the main part of the karitene precipitates, and is subsequently disposed of. The solvent is distilled off leaving a partly dekaritenised shea butter.
4. In the following fractionation process the fat is mixed with hexane and heated to transparency followed by a cooling designed to crystallise the high-melting fraction known under the INN-designation "Shea Stearine".
5. The Shea Stearine is filtered off and the filtrate desolventised leaving a low-melting, partly dekaritenised shea butter fraction. After post refining in the form of an alkaline deacidification and a subsequent treatment with bleaching earth the fraction is named PR 589.

The shea butter fraction PR 589 is characterised by the following typical values vs. shea butter:

| Parameter | Shea fraction PR 589 | Refined Shea |
|---|---|---|
| Butter | | |
| Slip melting point, °C | 20 | 32 |
| Acid value | 0.1 | 0.3 |
| Iodine value | 74 | 62 |
| Total unsaponifiables, % | 12.5 | 6 |
| Polyisoprenic hydrocarbons, % | 0.8 | 2 |

The shea butter fraction was fully hydrogenated to a slip melting point of 68 °C by the following process:
To 2500 g of the fraction was added 0.5% catalyst ("Girdler G-53", Süd-Chemie AG) and the mixture was heated to 180 °C. Hydrogen was applied at 5 ato for 160 minutes. The catalyst was filtered off, and the hydrogenated oil was denickled in the normal way. To the hydrogenated oil was added 10% by weight of shea butter fraction PR 589 to produce an oil mixture ready for fractionation. In the same manner another oil mixture was made by adding 20% of shea butter fraction PR 589 to the hydrogenated oil.

In jacketed vessels equipped with stirrer and containing 1000 g each of the two oil mixtures hexane was added in a ratio oil to hexane of 1:10 w/v and the mixture was heated to transparency. From a temperature of 36 °C the mixture was cooled at a rate of approx. 1 °C/min to reach a final temperature of 0 °C. The precipitated high-melting fractions are filtered off and the filtrate desolventised leaving two fractions rich in unsaponifiable matter. The two fractions were obtained in a yield of 21.0% and 29.0%, respectively.

The total mass of unsaponifiables in the oil mixture is 125g (12.5% of 1000 g). As can be seen in the following table the major part is recovered by the process.

| Parameter | Shea fraction | Fraction I | Fraction II |
|---|---|---|---|
| | PR-589 | (10%PR-589) | (20%PR-589) |
| Total unsaponifiables, % | 12.5 | 53.0 | 38.1 |
| Total trans-fatty acids, % | bdl | 0.7 | 0.4 |
| Enrichment factor calc. on | | | |
| total unsaponifiables | | 4.2 | 3.0 |
| Yield of fraction in g | | 210 | 290 |
| Recovered amount of | | | |
| unsaponifiables in g | | 111.3 | 110.5 |
| Recovery in % of total matter | | 89.0 | 88.4 |

The two fractions have a remarkably high content of unsaponifiable matter making them suited for a number of food and non-food applications. Especially in Fraction I the concentration has reached a level that makes it possible to use it in e.g. gelatine capsules for oral administration.

### Example 4. Industrial scale test-production of a fraction of shea butter enriched in unsaponifiable matter

In order to verify the applicability of the process on an industrial scale a fraction similar to Fraction I in Example 3 was produced as described in the following:
Shea butter fraction PR 589 was hydrogenated as described in Example 3. To 30 metric ton of the hydrogenated oil was added 4.5 metric ton of shea butter fraction PR 589.

To the oil mixture containing 13% carrier oil was added hexane in the ratio 1:10 v/v, and the mixture was heated to transparency and fractionated by cooling the mixture to reach a final temperature of 0 °C. The precipitated high-melting fraction was filtered off and the filtrate desolventised leaving a fraction rich in unsaponifiable matter in a yield of 23.5 % based on the weight of the oil mixture.

The recovered fraction was passed through a 50 µm filter and further purified by deodorisation at 220 °C and 2 mbar. After cooling to 100 °C 25 ppm citric acid and 500 ppm natural mixed tocopherols were added to the product to improve the oxidative resistance.

The final product has the following characteristics:

| Appearance | Yellowish, homogeneous paste |
|---|---|
| Slip melting point, °C | 28.6 |
| Solid fat content at 20 °C, % | 10.3 |
| Solid fat content at 30 °C, % | 7.1 |
| Solid fat content at 40 °C, % | 3.8 |
| Rancimat value, 120 °C, h | 31.7 |
| Acid value | 0.84 |
| Iodine value | 80.8 |
| Total unsaponifiable matter, % | 45.7 |

Fatty acid composition of the glyceridic part in %:

| | |
|---|---|
| C12:0 | 0.7 |
| C14:0 | 0.4 |
| C16:0 | 8.0 |
| C18:0 | 33.3 |
| C18:1 cis | 45.9 |
| C18:1 trans | 1.8 |
| C 18:2 cis | 7.0 |
| C18:2trans | 0.1 |
| C18:3 cis | 0.3 |
| C20:0 | 1.3 |
| C20:1 cis | 0.3 |
| C22:0 | 0.2 |
| Others | 0.7 |

The high-melting fraction was of a very good quality which makes it suited as a raw material for the production of food ingredients.

This example demonstrates the applicability of the invented process on an industrial scale.

### Example 5. Interesterified fraction of shea butter enriched in unsaponifiable matter

In shea butter the major part of the unsaponifiable matter is α- and β-amyrin, butyrospermol and lupeol, present in the form of cinnamic and acetic acid esters and to a lesser extent as fatty acid esters and the free alcohols. A minor part is the phytosterols i.e. α-spinasterol, stigmasterol, etc.

In order to increase the oil solubility of the unsaponifiables, the starting oil is interesterified before hydrogenation for the purpose of increasing the presence of fatty acid ester types. Shea butter fraction PR 589 is interesterified at a temperature of 120 °C with 0.1% sodium methoxide as a catalyst. The resulting interesterified product is named shea butter fraction PO 135. The relative composition and the total content of the unsaponifiable matter is given below:

| Constituents | | PR 589 | PO 135 |
|---|---|---|---|
| Total unsaponifiable content comprising: | | 12.5% | 12.0% |
| | TTPA-cinnamic esters | 50% | 48% |
| | TTPA-acetic esters | 22% | 21% |
| | TTPA-fatty acid esters | 4% | 14% |
| | TTPA and Phytosterols | 17% | 9% |
| | Karitene | 7% | 8% |

The shea butter fraction PO 135 is fully hydrogenated according to the procedure described in Example 3. An oil mixture is prepared by mixing the fully hydrogenated and the unhydrogenated shea butter fraction PO 135 in the ratio 1:1 by weight.

In order to randomise the glycerides the oil mixture is interesterified at a temperature of 120 °C for 15 minutes with 0.1% sodium methoxide as a catalyst. After treatment with citric acid and bleaching earth the resulting product is named shea butter fraction PO 136.

Shea butter fraction PO 136 is fractionated in two steps by mixing it with hexane in the ratio 1:4 (w/v) and heating the mixture to transparency and cooling it to 0 °C to precipitate the first high-melting fraction and filtering it off. The filtrate is distilled until the oil to hexane ratio is 1:4 (w/v). The second high-melting fraction is disposed of after cooling to -15 °C. The filtrate is desolventised leaving a fraction named PO 136EE.

Shea butter fraction PO 136EE has a total unsaponifiable content of 34.0%. The product has the following composition in which "TTPA" comprises hydrogenated moieties:

| | |
|---|---|
| Total glycerides | 66.0% |
| TTPA-acetic acid esters | 6.2% |
| TTPA-fatty acid esters | 7.5% |
| TTPA-cinnamic acid esters | 15.7% |
| Karitene | 2.0% |
| Other unsaponifiable matter | 2.6% |

Fatty acid composition of the glyceridic part:

| | |
|---|---|
| C14:0 | 0.1% |
| C16:0 | 4.0% |
| C16:1 cis | 0.1% |
| C18:0 | 29.6% |
| C18:1 cis | 54.4% |
| C18:1 trans | 0.6% |
| C18:2 cis | 9.4% |
| C18:2 trans | 0.0% |
| C18:3 cis | 0.3% |
| C20:0 | 0.8% |
| C20:1 cis | 0.5% |
| C22:0 | 0.1% |
| C22:1 cis | 0.1 % |

Shea butter fraction PO 136EE is tailored to be used for the nutritional fortification of any food product. Furthermore, it is especially suited to be used as an ingredient in fat-containing nutritional products for the purpose of lowering the blood cholesterol content.

If a higher degree of enrichment is needed for the intended use, this can be achieved by the fractionation of an oil mixture containing less unhydrogenated shea butter fraction PO 135 cf. Example 3.

### Example 6. Use of an oil, enriched with unsaponifiable matter originating from rapeseed, in a cosmetic formulation

From literature *(*British Journal of Dermatology 134, 215-220 (1996)) it is known that unsaponifiables from rapeseed have a beneficial effect in restoring the barrier function on surfactant-irritated skin.

In the following formulation the oil fraction enriched in rapeseed unsaponifiables from Example 2, where 5% "Cremeol PS-6" is added as carrier oil, is incorporated in a hand cream intended to be used after manual cleaning.

| Phase | Ingredient | CTFA/INCI Name | % w/w |
|---|---|---|---|
| A: | "Cremeol PS-6" | Vegetable Oil (and) | 6.00 |
| | enriched in rape- | Rapeseed (Brassica | |
| | seed unsaponi- | Campestris) Oil | |
| | fiables | Unsaponifiables | |
| | "Cremeol FR-36" | Glyceryl Dioleate | 6.00 |
| | "Tegin SE" | Glyceryl Stearate SE | 4.50 |
| | (Th Goldschmidt) | | |
| | "Cetiol 868" | Octyl Stearate | 2.00 |
| | (Cognis) | | |
| | "Cremeol HF-52" | Hydrogenated | 1.00 |
| | | Vegetable Oil | |
| | "Abil 100" | Dimethicone | 0.50 |
| | (Th Goldschmidt) | | |
| B: | Water, deionized | Water | ad 100.00 |
| | Glycerin, 99.5% | Glycerin | 4.00 |
| C: | Perfume and | | q.s. |
| | preservatives | | |

The product is produced by heating A and B to 75-80 °C while stirring and combining the two phases. The mixture is homogenised at 70 °C. Cool slowly to 30 °C while stirring. Add C and homogenise at 30 °C. The final viscosity of the emulsion is obtained after 2-3 days at room temperature.

### Example 7. Use of a fraction of sheanut oil, enriched in unsaponifiable matter, in cosmetic and pharma-like ointments

The use of fraction II in Example 3 in two waterless formulations intended to protect and restore the skin is illustrated in the following way:

### I. Lipbalm in stick form

| Ingredient | CTFA/INCI Name | % w/w |
|---|---|---|
| Shea fraction II | Shea Butter (Butyrospermum Parkii) Extract | 2.00 |
| "Cremeol VP" | Veg. Oil (and) Hydrogenated Veg. Oil (and) Candelilla (Euphorbia Cerifera) Wax | 73.40 |
| "Cremeol HF-52" | Hydrogenated Veg. Oil | 13.00 |
| Beeswax | Beeswax | 7.80 |
| Carnauba wax | Carnauba (Copemicia Cerifera) Wax | 3.80 |
| Fragrance | | q.s. |

All the ingredients are heated to 70-80 °C and mixed. The mixture is filled into moulds or 5 ml tubes at 65-70 °C and cooled.

The formulation was tested at various concentrations of shea fraction II by five female panelists skilled as daily users of lipbalm. A dosage of 2-3% was preferred in emolliency and incorporating an increase of the lasting effect by one to two hours. At higher concentrations the product was too sticky and declined as being a cosmetic product.

### II. Pharma-like Ointment

| Ingredient | % w/w |
|---|---|
| Shea fraction II | 20 |
| White Petrolatum, NF19 | 72 |
| White Wax, NF19 | 3 |
| Isopropyl palmitate | 5 |

All the ingredients are heated to 70-80 °C and mixed. The mixture is cooled while stirring. When the product starts to congeal it is filled into tubes. The ointment has a slip melting point of 47.6 °C and passed a storage test at 40 °C.

Due to the restoring, cicatrising and bacteriostatic properties of the sheanut oil unsaponifiable matter the product is intended for use on extremely dry, sensitive skins, wounds and various scars.

### Example 8. Use of a fraction of sheanut oil, enriched in unsaponifiable matter, as an active ingredient in a soft gelatine capsule for oral administration

Due to the high content of unsaponifiable matter in fraction I from Example 3 it is suited for encapsulation.

A mixture was made according to the following formulation:

| Ingredient | % w/w |
|---|---|
| Shea fraction I | 80% |
| "Shoguwar 41 NG/NF" (NF 19, hydrogen. veg. oil - Type II) | 20% |

"Shoguwar 41 NG/NF" is a soybean oil hydrogenated to a slip melting point of 41 °C. At 20 °C SFC is approx. 75% and at 37 °C SFC is approx. 20%. This makes it a good processing agent and carrier that will keep the active material suspended and uniformly distributed.

The two ingredients are dosed, melted and mixed at 45 °C and fed to the filling machine to produce soft gelatine capsules of 250 mg content.

A daily dose of 750 mg (250 mg three times daily) is equivalent to ca. 300 mg unsaponifiables.

### Example 9. A clinical study of the efficacy of an interesterified fraction of sheanut oil enriched in non-tocolic, high-melting unsaponifiable matter on plasma lipids and lipoproteins in healthy subjects with normal to borderline high plasma cholesterol

- **Setting:**: Aalborg Sygehus, Hobrovej 18, 9000 Aalborg, Denmark.
- **Project manager:**: Erik Berg Schmidt MD, D.M. Sci., Department of Medicine, Hjorring/Bronderslev Hospital, Hjorring, Denmark.
- **Project team:**: Inge Aardestrup, Department of Clinical Biochemistry, Aalborg Hospital, Aalborg, Denmark;
Jens Mellerup, R&D Department of Aarhus Oliefabrik A/S, Aarhus, Denmark;
Jeppe Hagstrup Christensen, Department of Nephrology, Aalborg Hospital, Aalborg, Denmark.

### Background

Plasma total cholesterol and low-density lipoprotein (LDL) cholesterol levels are strongly related to the risk of coronary heart disease (CHD), the major cause of premature death in Western societies. Thus, a reduction in total cholesterol of 1 % is associated with a 2 - 3 % decrease in the risk of CHD. A reduction of plasma cholesterol levels is therefore of paramount importance both for the individual patients and for the whole community. Intake of plant sterols can lower the plasma cholesterol concentrations in humans. However, little is known about the effects of the unsaponifiable constituents of sheanut oil, mainly the triterpene alcohols.

### Objective

To study the effect of a sheanut oil fraction, prepared according to Example 5, on plasma lipids and lipoproteins in healthy subjects with normal to borderline high plasma cholesterol.

### Design and Treatment

One hundred and five healthy volunteers (54 men of age 38.8 ± 13.4 y, range 20 - 64 y, and 51 women of age 40.7 + 12.6 y, range 23 - 60 y) were randomly assigned to 2 treatment groups in a double-blind study design. The subjects were randomized into treatment blocks of 16 persons in order to secure an equal distribution of men and women in the two groups. The Active group consumed 30 g/d of a sheanut oil spread, and the Control group consumed 30 g/d of a sunflower oil spread. A dietary questionnaire was filled out, and the subjects were asked to maintain their habitual diet during the whole study period. The subjects consumed one unit of spread 3 times a day and usually on a slice of bread, at breakfast, lunch, and supper. The diets were given for 6 consecutive weeks.

The R&D Department, Aarhus Oliefabrik A/S, Denmark, specially prepared the experimental spreads. They were packed in blinded foil envelopes each holding 10 g and labeled with a four-digit code. The fat phase of both spreads contained liquid oil and hard stock. The hard stock and the sunflower oil used in the two spreads came from the same batches. The liquid sheanut oil used, containing 33.2 % by weight of non-glyceridic constituents, was modified by fractionation, hydrogenation, and interesterification according to the procedure described in Example 5.

The sheanut oil spread contained 10 % non-glycerides, i.e. triterpene acetic acid esters, triterpene cinnamic acid esters, triterpene fatty acid esters, free triterpene alcohols, free sterols, and kariten (a naturally occurring polyisoprenic hydrocarbon in sheanuts). The sunflower oil spread was similar to the range of well-known sunflower oil products with a long history of heart health promotion.

Both spreads showed normal appearance and had almost identical structure. Compositions are shown in Table 1.

**TABLE 1**

| Composition of the experimental spreads. | | | | | | |
|---|---|---|---|---|---|---|
| | Sunflower | | | Sheanut | | |
| Body Fat 62¹ (g/100 g) | 19.66³ | | | 19.66³ | | |
| Solex² (g/100 g) | 58.98⁴ | | | 29.17⁴ | | |
| Sheanut oil (g/100 g) | | | | 29.81 | | |
| Fat soluble ingredients (g/100 g) | 1.36 | | | 1.36 | | |
| Water phase (g/100 g) | 20.00 | | | 20.00 | | |
| Saturated fatty acids (wt % in the glycerides): | | 25.9 | | | 34.4 | |
| - Lauric (C12:0) | | | 4.0 | | | 4.6 |
| - Myristic (C14:0) | | | 1.8 | | | 2.1 |
| -Palmitic (C16:0) | | | 15.6 | | | 16.3 |
| - Stearic (C 18:0) | | | 4.1 | | | 11.0 |
| - Arachidic (C20:0) | | | 0.4 | | | 0.4 |
| Monounsaturated fatty acids (wt % in the glycerides): | | 22.5 | | | 31.9 | |
| - Palmitoleic (C16:1, 9c) | | | 0.1 | | | 0.1 |
| -Oleic (C18:1, 9c) | | | 22.2 | | | 31.6 |
| - Gadoleic (C20:1, 9c) | | | 0.2 | | | 0.2 |
| Polyunsaturated fatty acids (wt % in the glycerides): | | 50.2 | | | 31.9 | |
| - Linoleic (C18:2, 9c, 12c) | | | 49.9 | | | 31.7 |
| - α-Linolenic (C18:3, 9c, 12c, 15c) | | | 0.3 | | | 0.2 |
| Trans fatty acids (wt % in the glycerides) | | 0.4 | | | 0.7 | |
| Non-glycerides (wt % of the fat phase): | | 1.1 | | | 12.6 | |
| - Triterpene acetic acid esters | | | | | | 2.2 |
| -Triterpene cinnamic acid esters | | | | | | 5.9 |
| - Triterpene fatty acid esters | | | | | | 3.5 |
| - Free triterpene alcohols and sterols | | | 0.3 | | | 0.3 |
| -Kariten⁵ | | | | | | 0.7 |

| | | | | | | |
|---|---|---|---|---|---|---|
| ¹Body Fat 62 (supplied by Aarhus Oliefabrik A/S) is the trade name of unhydrogenated vegetable hard stock for production of margarines and spreads. ²Solex (supplied by Aarhus Oliefabrik A/S) is the trade name of fully refined sunflower seed oil. ³From the same batch of Body Fat 62. ⁴From the same batch of Solex. ⁵A naturally occurring polyisoprenic hydrocarbon in sheanuts. | | | | | | |

### Results

The preparations were well tolerated, and all of the one hundred and five volunteers who started also completed the trial. Fifty-three subjects were allocated to the Active group, and 52 subjects participated in the Control group. Baseline characteristics of the study population are given in Table 2. Pre-experimentally no significant differences in the plasma cholesterol levels existed between the Active group and the Control group.

**TABLE 2**

| Characteristics of the volunteers | | | | |
|---|---|---|---|---|
| | Men (*n=54*) | | Women (*n=51*) | |
| | mean± s.d. | range | mean± s.d. | range |
| Age (y) | 38.8±13.4 | 20-64 | 40.7±12.6 | 23-60 |
| Total-cholesterol (mmol/L) | 5.04±1.22 | 2.80-8.30 | 5.15±1.01 | 3.45 - 7.85 |
| HDL-cholesterol (mmol/L) | 1.31±0.25 | 0.80-2.00 | 1.63 ± 0.32¹ | 0.80-2.40 |
| LDL-cholesterol mmol/L) | 3.18± 1.14² | 1.35-6.40 | 3.00±0.96 | 1.60-5.75 |

| | | | | |
|---|---|---|---|---|
| ¹Significantly different from men: P < 0.001. ² n = 53 | | | | |

There were significant reductions in plasma total- and LDL-cholesterol of 5 and 8 %, respectively, after treatment in the Active group.

The mean plasma linoleic acid concentration increased significantly by 6 % after the sheanut diet and significantly by 10 % after the sunflower diet. The mean serum stearic acid concentration increased significantly by 4 % after the sheanut diet and non-significantly by 1 % after the sunflower diet. These results are in the directions expected from the fatty acid contents of the diets and validate the participants' compliance to the dietary instructions.

The effect of the diets on plasma lipids and lipoproteins are given in Table 3 and the safety data are given in Table 4.

**TABLE 3**

| Plasma lipid and lipoprotein concentrations and physical data before inclusion and at the end of the six weeks' study period and the effects of the experimental diets. | | | | | | |
|---|---|---|---|---|---|---|
| | Sunflower diet *(n=52)* | | Sheanut diet *(n=53)* | | Effect of diets | |
| | Before | After | Before | After | Sunflower | Sheanut |
| Total-cholesterol, all ^{a} | 4.97±1.02 | 4.87 ± 1.00¹ | 5.22 ± 1.21 | 4.96 ± 1.08 ³ | -0.10 ± 0.32 | -0.25 ± 0.41 ^{*} |
| Total-cholesterol, men | 4.98 ± 1.10 ^{d} | 4.84 ± 1.06 | 5.10 ± 1.34 ^{e} | 4.85 ± 1.23 ² | -0.13 ± 0.36 ⁻ | -0.25 ± 0.39 |
| Total-cholesterol, women | 4.96 ± 0.95 ^{d} | 4.90 ± 0.95 | 5.35 ± 1.05 ^{c} | 5.09 ± 0.90² | -0.06± 0.28 | -0.26± 0.44 |
| HDL-cholesterol, all ^{a} | 1.48 ± 0.36 | 1.52 ± 0.36 | 1.45 ± 0.30 | 1.46 ± 0.32 | 0.04 ± 0.15 | 0.02 ± 0.13 |
| HDL-cholesterol, men | 1.31 ± 0.25 ^{d} | 1.35 ± 0.25 | 1.31 ± 0.26 ^{e} | 1.30 ± 0.26 | 0.04 ± 0.15 | -0.01 ± 0.11 |
| HDL-cholesterol, women | 1.66 ± 0.37 ^{d} | 1.69 ± 0.37 | 1.60 ± 0.28 ^{c} | 1.64 ± 0.29 | 0.03 ± 0.16 | -0.04 ± 0.14 |
| LDL-cholesterol, all ^{a} | 2.97 ± 0.97 ^{g} | 2.86 ± 0.88 ² | 3.21 ± 1.13 | 2.96 ± 0.97 ³ | -0.11 ± 0.29 | -0.25 ± 0.37^{*} |
| LDL-cholesterol, men | 3.15 ± 1.09^{c} | 3.00 ± 0.97 ¹ | 3.21 ± 1.21 ^{e} | 3.00 ± 1.07 ² | -0.15 ± 0.35 | -0.22 ± 0.34 |
| LDL-cholesterol, women | 2.81 ± 0.84 ^{d} | 2.73 ± 0.79 | 3.20 ± 1.05 ^{c} | 2.92 ± 0.86 ² | -0.08 ± 0.22 | -0.28 ± 0.41^{*} |
| Apolipoprotein B (g/L) | 0.92 ± 0.26 | 0.88 ± 0.23 ² | 1.00 ± 0.28 | 0.94 ± 0.23 ³ | -0.04 ± 0.08 | -0.07 ± 0.10 |
| Lipoprotein(a) (units/L) | 251.5 ± 338.4 ^{g} | 222.8 ± 288.6 ² | 286.2 ± 358.9 ^{f} | 264.9 ± 342.5 | -28.7 t 69.3 | -21.4 ± 80.5 |
| Triglycerides in serum ^{b} | 1.17 ± 0.92 | 1.15 ± 0.95 | 1.23 ± 0.55 | 1.18 ± 0.54 | -0.02 ± 0.24 | -0.05±0.32 |
| LDL:HDL-cholesterol | 2.14 ± 0.94 ^{g} | 2.00 ± 0.80 ² | 2.33 ± 1.08 | 2.13 ± 0.90 ³ | -0.14 ± 0.30 | -0.20 ±0.36 |
| LDL:Apolipoprotein B | 3.19 ± 0.34 ^{g} | 3.21 ± 0.33 | 3.25 ± 0.34 | 3.17 ± 0.36 | 0.02 ± 0.22 | -0.07 ±0.28 |
| Body Mass Index (kg/m²) | 24.78 ± 3.38 | 24.74 ± 3.42 | 25.58 ± 4.37 | 25.43 ± 4.41 ² | -0.05 ± 0.47 | -0.15 ± 0.36 |
| Diastolic BP (mmHg) | 71.3 ± 8.8 | 69.7 ± 7.5 | 72.9 ± 8.0 | 71.2 ± 8.4 ² | -1.5 ± 6.3 | -1.6 ± 4.4 |
| Systolic BP (mm Hg) | 116.7 ± 11.7 | 114.4 ± 10.5¹ | 117.8 ± 10.8 | 114.0 ± 11.3 ³ | -2.3 ± 7.0 | -3.8 ± 7.1 |
| C18:0 (wt %) | 12.16 ± 1.10 | 12.26 ± 1.27 | 12.01 ± 1.12 | 12.46 ± 1.03 ³ | 0.09 ± 0.73 | 0.45 ± 0.68 ^{*} |
| C18:2ω6 (wt %) | 20.82±2.52 | 22.81 ± 2.66 ³ | 20.62 ± 2.13 | 21.84 ± 2.36 ³ | 1.99 ± 2.20 | 1.22 ± 1.89 |

| | | | | | | |
|---|---|---|---|---|---|---|
| ¹ Significantly different from "Before": P < 0.05; ² significantly different from "Before": P < 0.01; ³ significantly different from "Before": P < 0.001. ^{*} Significantly different from "Sunflower": P < 0.05. ^{a} In mmol/L. To convert total-, HDL-, and LDL-cholesterol values to mg/dL multiply by 38.67. ^{b} In mmol/L. To convert triglyceride values to mg/dL multiply by 88.54. ^{c}n=25; ^{d}n=26; ^{e}n=28; ^{f}n=49; ^{g}n=51. | | | | | | |

**TABLE 4**

| Safety data before inclusion and at the end of the six weeks' study period and the effects of the experimental diets. | | | | | | |
|---|---|---|---|---|---|---|
| | Sunflower diet *(n=52)* | | Sheanut diet *(n=53)* | | Effect of diets | |
| | Before | After | Before | After | Sunflower | Sheanut |
| Sodium (mmol/L) | 144.7±2.1 | 144.4±1.7 | 144.8±1.8 | 144.6±1.8 | -0.3±1.7 | -0.2±1.7 |
| Potassium (mmol/L) | 4.20 ± 0.21 | 4.14 ± 0.25 | 4.20 ± 0.18 | 4.17 ± 0.19 | -0.06 ± 0.24 | -0.03 ± 0.21 |
| Magnesium (mmol/L) | 0.815 ± 0.062 | 0.799 ± 0.055 ¹ | 0.817 ± 0.056 | 0.802 ± 0.061 ¹ | -0.015±0.052 | -0.015±0.041 |
| Iron (µmol/L) | 16.37±5.98 | 18.25 ±6.64 | 18.66 ± 5.96 | 18.49±6.71 | 1.88 ± 7.87 | -0.17±7.70 |
| Calcium ^{a} (mmol/L) | 2.468 ± 0.062 | 2.473 ± 0.058 | 2.456 ± 0.048 | 2.465 ± 0.055 | 0.005 ± 0.043 | 0.008 ± 0.050 |
| ASAT ^{b} (units/L) | 22.00 ± 7.92 | 22.88 ± 9.34 | 21.83 ± 5.57 | 22.17 ± 5.84 | 0.88 ± 9.91 | 0.34 ± 4.56 |
| Bilirubin (µmol/L) | 11.04 ± 3.06 | 11.94 ± 3.99 ¹ | 11.30 ± 3.46 | 10.89 ± 3.21 | 0.90 ± 3.24 | -0.42 ± 2.58 ^{*} |
| ALP ^{c} (units/L) | 137.8 ± 38.54 | 134.2 ± 38.99 | 131.5 ± 31.01 | 129.9 ± 29.15 | -3.63 ± 14.45 | -1.62 ± 13.63 |
| Vitamine B12 (pmol/L) | 267.4 ± 114.1 | 258.2 ± 121.5 | 232.6 ± 83.3 | 224.0 ± 88.9 | -9.2 ± 45.1 | -8.6 ± 43.6 |
| INR ^{d} | 1.05 ± 0.09 | 1.06 ± 0.09 | 1.06 ± 0.09 | 1.05 ± 0.08 | 0.00 ± 0.04 | -0.01 ± 0.05 |
| Haemoglobin (mmol/L) | 8.360 ± 0.665 | 8.371 ± 0.699 | 8.549 ± 0.750 | 8.534 ± 0.699 | 0.012 ± 0.341 | -0.015 ± 0.373 |
| Leucocytes (10⁹/L) | 5.53 ± 1.81 | 5.56 ± 1.54 | 5.59 ± 1.39 | 5.86 ± 1.62 ¹ | 0.03 ± 1.15 | 0.27 ± 0.98 |
| Trombocytes (10⁹/L) | 236.4 ± 51.80 | 228.5 ± 53.97 ² | 241.0 ± 54.98 | 237.8 ± 48.16 | -7.88 ± 19.58 | -3.23 ± 25.26 |

| | | | | | | |
|---|---|---|---|---|---|---|
| ¹ Significantly different from "Before": P < 0.05; ² significantly different from "Before": P < 0.01. ^{*} Significantly different from "Sunflower": P < 0.05. ^{a} Adjusted for albumin. ^{b} Aspartate aminotransferase; ^{c} alkaline phosphatase; ^{d} International Normalized Ratio. | | | | | | |

### Conclusion

In summary the study shows that consumption of a sheanut oil spread containing unsaponifiable material, which is a mixture of triterpene alcohols such as butyrospermol, α-amyrin, β-amyrin, lupeol, and germanicol, is effective and safe in lowering plasma total- and LDL-cholesterol levels in healthy adults. The observed reduction in LDL-cholesterol in the present study is of clinical relevance, and in the order of magnitude of what is commonly seen in subjects given dietary advice for hypercholesterolemia. We therefore conclude that intake of this type of sheanut oil can make a contribution to the prevention of CHD.

## Claims

1. A process for preparing a vegetable oil fraction rich in non-tocolic, high-melting, unsaponifiable matter which comprises the following steps:
a) a vegetable oil, or a fraction thereof, having a slip melting point of not more than 30 °C and a content of unsaponifiable matter of at least 0.5 % by weight is hydrogenated to fully saturate the fatty acids of the glycerides and to reach a slip melting point of at least 57 °C;
b) to the hydrogenated oil is added from 1 to 75 % by weight of the unhydrogenated starting oil or another oil having a slip melting point of max. 30 °C to act as a carrier and vehicle for the unsaponifiable matter;
c) to the oil mixture from b) is added a solvent in a ratio from 1:2 to 1:20, and the mixture is heated to transparency;
d) the mixture from c) is cooled in one or more steps to a final temperature in the range from -35 to +30 °C, and the precipitated high-melting fraction(s) is (are) filtered off;
e) the filtrate obtained from d) is desolventised, leaving a fraction rich in unsaponifiable matter.

2. A process according to claim 1, wherein from 2 to 50 % by weight, and preferably from 5 to 25 % by weight of carrier is added to the hydrogenated oil in step b).

3. A process according to claim 1 or 2, wherein unhydrogenated starting oil is used as the carrier in step b).

4. A process according to claim 1 or 2, wherein a triglyceride oil different from the unhydrogenated starting oil is used as the carrier in step b), said triglyceride oil predominantly consisting of triglycerides of saturated and unsaturated C8-C22 fatty acids, and preferably being a vegetable oil predominantly consisting of triglycerides of saturated and unsaturated C16-C22 fatty acids.

5. A process according to any one of the preceding claims, wherein the starting oil containing the unsaponifiable matter is interesterified before the hydrogenation step in order to increase the oil solubility of a part of the unsaponifiables.

6. A process according to any one of the preceding claims, wherein the oil mixture from step b) is interesterified before the fractionation steps c) and d) in order to randomise the fatty acid content and incorporate unsaturated fatty acid in the glyceridic part of the resulting enriched oil fraction.

7. A process according to claim 5 or 6, wherein the interesterification is performed for at least 5 minutes in the presence of a suitable catalyst.

8. A process according claim 7, wherein the catalyst is selected from the group consisting of methoxide, ethoxide, and a mixture of glycerol and caustic lye.

9. A process according to any one of the preceding claims, wherein the solvent used in the fractionation steps c) and d) is of a non-polar or semi-polar type.

10. A process according claim 9, wherein the solvent is selected from the group consisting of hydrocarbons and linear and branched alkanols of 1-5 carbon atoms, and is preferably hexane or petroleum ether.

11. A process according to any one of the preceding claims, wherein the starting vegetable oil containing the unsaponifiable matter originates from one of the following or their hybrids: rapeseed, canola, soybean, corn, maize-germ, sunflower, low-linolenic linseed, mango, avocado, olive, sesame, rice bran, wheat germ, oat and oat bran, palm, sal, shorea, illipe and shea, as well as any fraction or mixture thereof.

12. A process according to any one of the preceding claims, wherein the starting vegetable oil is a dekaritenised, lower-melting fraction of shea butter enriched in unsaponifiable matter, which is obtained as follows:
The crude shea butter is deacidified and subsequently partly dekaritenised by mixing it with a semi-polar solvent to precipitate the major part of the polyisoprenic hydrocarbons, viz. karitene which are disposed of; the solvent is distilled off and the oil is mixed with a suitable solvent e.g. hexane (ratio approx. 1:2 to 1:4), heated to transparency, and cooled to a temperature at which the high-melting glycerides precipitate; the precipitated fraction is filtered off and the filtrate desolventised leaving a lower-melting fraction with a slip melting point of max. 30 °C suitable for further processing.

13. A process according to any one of the preceding claims, wherein the vegetable oil fraction obtained is further subjected to concentration by means of supercritical fluid extraction, molecular distillation, chromatography or recrystallisation.

14. A process according to any one of the preceding claims, wherein the vegetable oil fraction obtained is further subjected to chemical modification such as hydrogenation, ethoxylation, propoxylation, esterification or interesterification.

15. A process according to any one of the preceding claims, wherein the physical state of the vegetable oil fraction obtained is further changed e.g. by emulsification, incorporation in liposomes or nanosomes, admixing with a high-melting fat and spray cooling, or microencapsulation.

16. A vegetable oil fraction obtainable by the process according to any one of claims 1-15, said fraction being enriched in non-tocolic, high-melting, unsaponifiable matter by a factor of at least 3 and having a trans-fatty acid content of maximum 2% by weight.

17. A vegetable oil fraction according to claim 16, wherein the unsaponifiable matter is comprised of or derived from:
- carotenoids;
- sesamin and sesamolin;
- γ-oryzanol;
- sterols, methyl sterols and dimethyl sterols;
- triterpene alcohols and their esters with cinnamic acid, acetic acid and fatty acids.

18. A shea butter fraction obtainable by the process according to any one of claims 1-15, having a content of at least 30 % by weight of non-tocolic, high-melting, unsaponifiable matter and being further **characterised by** containing hydrogenated triterpene alcohols and/or their esters and by having a trans-fatty acid content of maximum 2 % by weight.

19. A shea butter fraction according to claim 18 having a content of 40-90 % by weight of said unsaponifiable matter.

20. A shea butter fraction according to claim 18 or 19, obtained by the process according to any one of claims 5-15, i.e. involving at least one interesterification step before the fractionation steps c) and d).

21. A vegetable oil fraction according to any one of claims 16-17 for use as an ingredient in pharmaceuticals or pharma-like products for topical application or oral administration.

22. Non-therapeutical use of a vegetable oil fraction according to any one of claims 16-17 for the fortification of food products.

23. Use of a vegetable oil fraction according to any one of claims 16-17 as a constituent of a dairy or dairy-like food product selected from the group consisting of milk, cream, ice cream, butter, cheese, including soft, cream and processed cheeses, yoghurt and other fermented milk products.

24. Use of a vegetable oil fraction according to any one of claims 16-17 as a constituent of a food product selected from the group consisting of margarines, butters and blends, spreads, mayonnaise, shortenings, dressings and salad oils at a concentration of 2-50% by weight.

25. Non-therapeutical use of a vegetable oil fraction according to any one of claims 16-17 as an ingredient in cosmetics for topical application.

26. Non-therapeutical use of a vegetable oil fraction according to any one of claims 16-17 as an ingredient in nutritional supplements for oral administration.

27. Non-therapeutical use of a shea butter fraction according to any one of claims 18-20 as an ingredient in nutritional supplements.

28. Use of a shea butter fraction according to any one of claims 18-20 for the manufacture of a medicament for lowering the blood cholesterol level.

## Patentansprüche

1. Verfahren zur Herstellung einer Pflanzenölfraktion, die reich an nichttocolhaltigem, hochschmelzendem, unverseifbarem Material ist, welches die folgenden Schritte umfasst:
a) ein Pflanzenöl oder eine Fraktion davon, aufweisend einen Slip-Schmelzpunkt von nicht mehr als 30°C, und ein Inhalt von unverseifbarem Material von mindestens 0,5 Gew.-%, wird hydriert, um die Fettsäuren der Glyceride vollständig zu sättigen, und um einen Slip-Schmelzpunkt von mindestens 57°C zu erzielen;
b) es wird dem hydrierten Öl eine Zugabe von 1 bis 75 Gew.-% des unhydrierten Ausgangsöls oder eines anderen Öls erteilt, aufweisend einen Slip-Schmelzpunkt von max. 30° C, um als ein Träger und Vehikel für das unverseifbare Material zu funktioneren;
c) der Ölmischung aus b) wird ein Lösungsmittel in einem Verhältnis von 1:2 bis 1:20 zugegeben, und die Mischung wird zur Transparenz erhitzt;
d) die Mischung aus c) wird in einem oder mehreren Schritten auf eine Endtemperatur im Bereich von -35 bis +30 °C gekühlt, und die präzipitierte hochschmelzende Fraktion(en) wird (werden) abgefiltert;
e) das aus d) erhaltene Filtrat wird desolventisiert, wodurch eine an unverseifbarem Material reiche Fraktion zurückgelassen wird.

2. Verfahren nach Anspruch 1, wobei dem hydrierten Öl in Schritt b) eine Zugabe von 2 bis 50 Gew.-% und bevorzugt von 5 bis 25 Gew.-% eines Trägers erteilt wird.

3. Verfahren nach Anspruch 1 oder 2, wobei unhydriertes Ausgangsöl als der Träger in Schritt b) verwendet wird.

4. Verfahren nach Anspruch 1 oder 2, wobei ein Triglyceridöl anders als das unhydrierte Ausgangsöl als der Träger in Schritt b) verwendet wird, welches Triglyceridöl überwiegend aus Triglyceriden von gesättigten und ungesättigten C8-C22-Fettsäuren besteht und bevorzugt ein Pflanzenöl ist, überwiegend bestehend aus Triglyceriden von gesättigten und ungesättigten C16-C22-Fettsäuren.

5. Verfahren nach irgendeinem der vorhergehenden Ansprüche, wobei das das unverseifbare Material enthaltende Ausgangsöl vor dem Hydrierungsschritt umgeestert wird, um die Öllöslichkeit eines Teils der unverseifbaren Stoffen zu erhöhen.

6. Verfahren nach irgendeinem der vorhergehenden Ansprüche, wobei die Ölmischung aus Schritt b) vor dem Fraktionierungsschritt c) und d) umgeestert wird, um den Fettsäureinhalt zu randomisieren, und eine ungesättigte Fettsäure in den glyceridischen Teil der resultierenden angereicherten Ölfraktion einzuarbeiten.

7. Verfahren nach Anspruch 5 oder 6, wobei die Umesterung in der Gegenwart von einem geeigneten Katalysator für mindestens fünf Minuten durchgeführt wird.

8. Verfahren nach Anspruch 7, wobei der Katalysator aus der Gruppe ausgewählt wird, bestehend aus Methoxid, Ethoxid, und einer Mischung von Glycerol und kaustischer Lauge.

9. Verfahren nach irgendeinem der vorhergehenden Ansprüche, wobei das im Fraktionierungsschritt c) und d) verwendete Lösungsmittel von einem nicht-polaren oder semi-polaren Typ ist.

10. Verfahren nach Anspruch 9, wobei das Lösungsmittel aus der Gruppe ausgewählt wird, bestehend aus Kohlenwasserstoffen und linearen und verzweigten Alkanolen von 1-5 Kohlenstoffatomen, und bevorzugt Hexan oder Erdölether ist.

11. Verfahren nach irgendeinem der vorhergehenden Ansprüche, wobei das das unverseifbare Material enthaltende Ausgangspflanzenöl aus einem der Folgenden oder deren Hybriden: Rapssaat, Canola, Sojabohne, Getreide, Maiskeim, Sonnenblume, Niedriglinolenleinsaat, Mango, Avocado, Oliven, Sesam, Reiskleie, Weizenkeim, Hafer und Haferkleie, Palm, Sal, Shorea, Illipe und Shea, sowie jeder Fraktion oder Mischung davon stammt.

12. Verfahren nach irgendeinem der vorhergehenden Ansprüche, wobei das Ausgangspflanzenöl eine dekaritenisierte, niedriger-schmelzende Fraktion von Sheabutter ist, angereichert an unverseifbarem Material, welches wie folgt erhalten wird:
Die grobe Sheabutter wird entsäuert und darauffolgend teilweise dekaritenisiert, indem sie mit einem semi-polaren Lösungsmittel gemischt wird, um den größeren Teil der polyisoprenischen Kohlenwasserstoffe, und zwar Karitene, zu präzipitieren, welche beseitigt werden; das Lösungsmittel wird abdestilliert, und das Öl wird mit einem geeignetem Lösungsmittel gemischt, z.B. Hexan (Verhältnis ungefähr 1:2 bis 1:4), zur Transparenz erhitzt und auf eine Temperatur gekühlt, bei der die hochschmelzenden Glyceride präzipitieren; die präzipitierte Fraktion wird abgefiltert und das Filtrat desolventisiert, wodurch eine niedriger-schmelzende Fraktion mit einem Slip-Schmelzpunkt von max 30°C zurückgelassen wird, geeignet für eine weitere Verarbeitung.

13. Verfahren nach irgendeinem der vorhergehenden Ansprüche, wobei die erhaltene Pflanzenölfraktion zusätzlich einer Konzentration mittels überkritischer Fluidextraktion, Molekulardestillation, Chromatographie oder Umkristallisierung unterworfen wird.

14. Verfahren nach irgendeinem der vorhergehenden Ansprüche, wobei die erhaltene Pflanzenölfraktion zusätzlich einer chemischen Modifikation wie Hydrierung, Ethoxylierung, Propoxylierung, Veresterung oder Umesterung unterworfen wird.

15. Verfahren nach irgendeinem der vorhergehenden Ansprüche, wobei der physikalische Zustand der erhaltenen Pflanzenölfraktion zusätzlich verändert wird, z.B. durch Emulgierung, Einarbeitung in Liposome oder Nanosome, Bemischung mit einem hochschmelzenden Fett und Sprühkühlung, oder Mikroverkapselung.

16. Pflanzenölfraktion, erhältlich durch das Verfahren nach irgendeinem der Ansprüche 1-15, welche Fraktion an nichttocolhaltigem, hochschmelzendem, unverseifbarem Material um einen Faktor von mindestens 3 angereichert ist und einen Trans-Fettsäureinhalt von maximal 2 Gew.-% aufweist.

17. Pflanzenölfraktion nach Anspruch 16, wobei das unverseifbare Material besteht aus oder ist abgeleitet aus:
- Carotinoiden;
- Sesamin und Sesamolin;
- γ-Oryzanol;
- Sterolen, Methylsterolen und Dimethylsterolen;
- Triterpenalkoholen und deren Estern mit Zimtsäure, Essigsäure und Fettsäuren.

18. Sheabutterfraktion, die durch das Verfahren nach irgendeinem der Ansprüche 1-15 erhältlich ist, welche einen Inhalt von mindestens 30 Gew.-% von nichttocolhaltigem, hochschmelzendem, unverseifbarem Material aufweist und zusätzlich **dadurch gekennzeichnet ist, dass** sie hydrierte Triterpenalkohole und/oder deren Ester enthält und einen Trans-Fettsäureinhalt von maximal 2 Gew.-% aufweist.

19. Sheabutterfraktion nach Anspruch 18, aufweisend einen Inhalt von 40-90 Gew.-% des unverseifbaren Materials.

20. Sheabutterfraktion nach Anspruch 18 oder 19, erhalten durch das Verfahren nach irgendeinem der Ansprüche 5-15, d.h. Einbeziehung mindestens eines Umesterungsschritts vor dem Fraktionierungsschritt c) und d).

21. Pflanzenölfraktion nach irgendeinem der Ansprüche 16-17 zur Verwendung als eine Ingredienz in pharmazeutischen Produkten oder pharmaähnlichen Produkten für topische Applikation oder orale Verabreichung.

22. Nicht-therapeutische Verwendung einer Pflanzenölfraktion nach irgendeinem der Ansprüche 16-17 zur Verstärkung von Nahrungsmittelprodukten.

23. Verwendung einer Pflanzenölfraktion nach irgendeinem der Ansprüche 16-17 als ein Bestandteil eines Milch- oder eines milchähnlichen Nahrungsmittelprodukts, ausgewählt aus der Gruppe, bestehend aus Milch, Rahm, Eiscreme, Butter, Käse, hierunter Weich-, Rahm- und verarbeiteten Käsen, Yoghurt und sonstigen fermentierten Milchprodukten.

24. Verwendung einer Pflanzenölfraktion nach irgendeinem der Ansprüche 16-17 als ein Bestandteil eines Nahrungsmittelprodukts, ausgewählt aus der Gruppe, bestehend aus Margarinen, Buttern und Vermischungen, Aufstrichen, Mayonnaise, Backfetten, Dressings und Salatölen bei einer Konzentration von 2-50 Gew.-%.

25. Nicht-therapeutische Verwendung einer Pflanzenölfraktion nach irgendeinem der Ansprüche 16-17 als eine Ingredienz in Kosmetika für topische Applikation.

26. Nicht-therapeutische Verwendung einer Pflanzenölfraktion nach irgendeinem der Ansprüche 16-17 als eine Ingredienz in Ernährungsergänzungen für orale Verabreichung.

27. Nicht-therapeutische Verwendung einer Sheabutterfraktion nach irgendeinem der Ansprüche 18-20 als eine Ingredienz in Ernährungsergänzungen.

28. Verwendung einer Sheabutterfraktion nach irgendeinem der Ansprüche 18-20 zur Herstellung eines Medikaments zur Senkung des Blutcholesterinspiegels.

## Revendications

1. Procédé pour la préparation d'une fraction d'huile végétale riche en matière non-tocolique, à point de fusion élevé et insaponifiable, qui comprend les étapes suivantes:
a) une huile végétale, ou une fraction de celle-ci, ayant un point de fusion de glissement inférieur à 30°C et une teneur en matière insaponifiable d'au moins 0,5 % en poids, est hydrogénée pour saturer complètement les acides gras des glycérides et pour atteindre un point de fusion de glissement d'au moins 57°C;
b) à l'huile hydrogénée, on ajoute de 1 à 75 % en poids de l'huile non hydrogénée de départ ou d'une autre huile ayant un point de fusion de glissement d'un maximum de 30°C pour agir en tant que porteur et véhicule pour la matière insaponifiable;
c) au mélange d'huile de l'étape b) est ajouté un solvant dans un ratio de 1:2 à 1:20, et le mélange est chauffé à la transparence;
d) le mélange de l'étape c) est refroidi dans une ou plusieurs étapes à une température finale comprise entre -35 à +30°C, et la fraction précipitée ou les fractions précipitées à point de fusion élevé est/sont filtrée(s);
e) le filtrat obtenu de l'étape d) est désolvantisé, laissant une fraction riche en matière insaponifiable.

2. Procédé selon la revendication 1, dans lequel 2 à 50 % en poids, et de préférence de 5 à 25% en poids de porteur est ajouté à l'huile hydrogénée à l'étape b).

3. Procédé selon la revendication 1 ou 2, dans lequel l'huile non hydrogénée de départ est utilisée en tant que porteur dans l'étape b).

4. Procédé selon la revendication 1 ou 2, dans lequel une huile triglycéride différente de l'huile non hydrogénée de départ est utilisée en tant que porteur à l'étape b), ladite huile triglycéride étant principalement composée de triglycérides des acides gras saturés et insaturés de C8-C22, et étant de préférence une huile végétale principalement composée de triglycérides des acides gras saturés et insaturés de C16-C22.

5. Procédé selon l'une quelconque des revendications précédentes, dans lequel l'huile de départ contenant la matière insaponifiable est interestérifiée avant l'étape d'hydrogénation afin d'augmenter la solubilité de l'huile d'une partie des insaponifiables.

6. Procédé selon l'une quelconque des revendications précédentes, dans lequel le mélange d'huile de l'étape b) est interestérifié avant les étapes de fractionnement c) et d), afin de randomiser la teneur en acides gras et d'incorporer les acides gras insaturés dans la partie glycéridique de la fraction résultante d'huile enrichie.

7. Procédé selon la revendication 5 ou 6, dans lequel l'interestérification est effectuée pendant au moins 5 minutes en présence d'un catalyseur approprié.

8. Procédé selon la revendication 7, dans lequel le catalyseur est choisi parmi le groupe constitué du méthoxyde, de l'éthoxyde, et d'un mélange de glycérine et de lessive caustique.

9. Procédé selon l'une quelconque des revendications précédentes, dans lequel le solvant utilisé dans les étapes de fractionnement c) et d) est d'un type non-polaire ou semi-polaire.

10. Procédé selon la revendication 9, dans lequel le solvant est choisi parmi le groupe constitué d'hydrocarbures et d'alcanols linéaires et ramifiés de 1 à 5 atomes de carbone, et est de préférence l'hexane ou l'éther de pétrole.

11. Procédé selon l'une quelconque des revendications précédentes, dans laquelle l'huile végétale de départ contenant la matière insaponifiable provient de l'un du groupe suivant ou de leurs hybrides: le colza, le canola, le soja, le maïs, des germes de maïs, le tournesol, des graines de lin linoléique bas, la mangue, l'avocat, l'olive, le sésame, le son de riz, les germes de blé, l'avoine et le son d'avoine, le palme, le sal, le shorea, l'illipé et le karité, ainsi que toute fraction ou tout mélange de ceux-ci.

12. Procédé selon l'une quelconque des revendications précédentes, dans laquelle l'huile végétale de départ est une fraction dékaritenisée, à point de fusion bas du beurre de karité et enrichie en matière insaponifiable, qui est obtenue comme suit:
Le beurre de karité brut est désacidifié et ensuite partiellement dékaritenisé en étant mélangé avec un solvant semi-polaire pour précipiter la plupart des hydrocarbures polyisopréniques, à savoir karitène, qui sont éliminés; le solvant est distillé et l'huile est mélangée avec un solvant approprié, par exemple l'hexane (ratio d'environ 1:2 à 1:4), chauffée à la transparence, et refroidie à une température à laquelle les glycérides à point de fusion élevé précipitent; la fraction précipitée est filtrée et le filtrat désolvantisé laissant une fraction à point de fusion bas avec un point de fusion de glissement d'un maximum de 30°C adaptés pour un traitement ultérieur.

13. Procédé selon l'une quelconque des revendications précédentes, dans lequel la fraction de l'huile végétale obtenue est en outre soumise à une concentration par des moyens de l'extraction par fluide supercritique, la distillation moléculaire, la chromatographie ou la recristallisation.

14. Procédé selon l'une quelconque des revendications précédentes, dans lequel la fraction de l'huile végétale obtenue est en outre soumise à des modifications chimiques, telles que l'hydrogénation, l'éthoxylation, la propoxylation, l'estérification ou l'interestérification.

15. Procédé selon l'une quelconque des revendications précédentes, dans lequel l'état physique de la fraction d'huile végétale obtenue est davantage modifié par exemple par l'émulsification, l'incorporation dans des liposomes ou nanosomes, le mélange avec une matière grasse à point de fusion élevé et le refroidissement par pulvérisation, ou la microencapsulation.

16. Fraction de l'huile végétale obtenue par le procédé selon une quelconque des revendications 1 à 15, ladite fraction étant enrichie en matière non tocolique, à point de fusion élevé et insaponifiable par un facteur d'au moins 3 et ayant une teneur en acide gras trans d'un maximum de 2 % en poids.

17. Fraction de l'huile végétale selon la revendication 16, dans laquelle la matière insaponifiable est composée ou proviennent:
- des caroténoïdes;
- de la sésamine et de la sésamoline;
- du gamma oryzanol;
- des stérols, des stérols de méthyle et des stérols de diméthyle;
- des alcools triterpéniques et de leurs esters avec l'acide cinnamique, l'acide acétique et des acides gras.

18. Fraction de beurre de karité obtenue par le procédé selon l'une quelconque des revendications 1 à 15, ayant une teneur d'au moins 30% en poids en matière non tocolique, à point de fusion élevé et insaponifiable et étant davantage **caractérisée par** leur teneur en alcools triterpéniques hydrogénés et/ou leurs esters et en ayant une teneur en acide gras trans d'un maximum de 2% en poids.

19. Fraction de beurre de karité selon la revendication 18, ayant une teneur de 40 à 90 % en poids de ladite matière insaponifiable.

20. Fraction de beurre de karité selon la revendication 18 ou 19, obtenue par le procédé selon l'une quelconque des revendications 5 à 15, c'est-à-dire impliquant au moins une étape de transestérification avant les étapes c) et d) de fractionnement.

21. Fraction de l'huile végétale selon l'une quelconque des revendications 16 à 17 à utiliser en tant qu'un ingrédient dans les produits pharmaceutiques ou les produits de type pharmaceutique pour application topique ou pour l'administration orale.

22. Utilisation non thérapeutique d'une fraction d'huile végétale selon l'une quelconque des revendications 16 à 17 pour la fortification des produits alimentaires.

23. Utilisation d'une fraction d'huile végétale selon l'une quelconque des revendications 16 à 17 en tant qu'un constituant d'une laiterie ou d'un produit laitier sélectionné parmi le groupe composé de lait, crème, crème glacée, beurre, fromage y compris fromages doux, à la crème et traités, yaourts et d'autres produits laitiers fermentés.

24. Utilisation d'une fraction d'huile végétale selon l'une quelconque des revendications 16 à 17 en tant qu'un constituant d'un produit alimentaire sélectionné parmi le groupe composé des margarines, des beurres et des mélanges, des pâtes à tartiner, de la mayonnaise, des shortenings, des sauces et des huiles à salade à une concentration de 2 à 50% en poids.

25. Utilisation non thérapeutique d'une fraction d'huile végétale selon l'une quelconque des revendications 16 à 17 en tant qu'un ingrédient dans les produits cosmétiques pour l'application topique.

26. Utilisation non thérapeutique d'une fraction d'huile végétale selon l'une quelconque des revendications 16 à 17 en tant qu'un ingrédient dans les suppléments nutritionnels pour l'administration orale.

27. Utilisation non thérapeutique d'une fraction de beurre de karité selon l'une quelconque des revendications 18 à 20 en tant qu'ingrédient dans les suppléments nutritionnels.

28. Utilisation d'une fraction de beurre de karité selon l'une quelconque des revendications 18 à 20 pour la fabrication d'un médicament pour abaisser le niveau de cholestérol sanguin.
